# FASCICULE DE BREVET EUROPEEN

(11) **EP 4 106 616 B1**
(45) Date de publication et mention de la délivrance du brevet: **08.04.2026**
(21) Numéro de dépôt: 21711298.6
(22) Date de dépôt: 18.02.2021
(51) Int. Cl.: A61B 5/00

(54) **DISPOSITIF DE MESURE DESTINÉ À ÊTRE PLACÉ AU CONTACT D'UN TISSU ET PROCÉDÉ D'ANALYSE DES DONNÉES TISSULAIRES MESURÉES**
MESSGERÄT ZUR KONTAKTIERUNG MIT EINEM GEWEBE UND VERFAHREN ZUR ANALYSE DER GEMESSENEN GEWEBEDATEN
MEASURING DEVICE INTENDED FOR BEING PLACED IN CONTACT WITH A TISSUE AND A METHOD FOR ANALYSING THE MEASURED TISSUE DATA

(30) Priorité: 20.02.2020 FR 2001710
(43) Date de publication de la demande: 28.12.2022
(73) Titulaire: Biomeca, 69007 Lyon (FR)
(72) Inventeur: CHLASTA, Julien, 69110 Sainte-Foy-lès-Lyon (FR)
(74) Mandataire: Germain Maureau
(86) Numéro de dépôt international: PCT/FR2021/050296
(87) Numéro de publication internationale: WO 2021/165624

(56) Documents cités:
- US-A1- 2013 291 658
- US-A1- 2017 231 499
- US-A1- 2019 029 592

## Description

### Domaine technique de l'invention

La présente invention concerne un dispositif de mesure destiné à être placé au contact d'un tissu.

L'invention concerne aussi un procédé d'analyse de données tissulaires.

### Etat de la technique

Dans le domaine de l'analyse des tissus biologiques, les méthodes d'analyses sont nombreuses et généralement dépendantes des tissus à analyser. Dans le cas de la peau, une méthode généralement utilisée pour les lésions supposées cancéreuses, est une analyse visuelle par un praticien. Cette méthode d'analyse repose sur la règle ABCDE, qui permet au praticien d'évaluer l'état d'une lésion cutanée selon des critères morphologiques tels que l'asymétrie, la régularité du bord, la couleur, le diamètre et l'épaisseur. Parfois, cette méthode est supportée par la dermoscopie.

Cependant, on constate dans la grande majorité des cas que l'expérience et la formation du praticien augmentent fortement la performance de l'analyse des données issues des tissus biologiques. La sensibilité et la spécificité de ces méthodes sont assez bonnes lorsque le praticien a une bonne connaissance de la sémiologie des lésions cutanées, ce qui n'est généralement pas le cas chez les médecins généralistes, ni chez les médecins spécialistes inexpérimentés.

Par ailleurs, malgré l'expérience du praticien, l'analyse des données issues des tissus biologiques peut être altérée par la nature de la peau analysée. En effet, les peaux humaines sont composées de trois couches : l'épiderme, le derme et l'hypoderme. L'épiderme est la couche la plus superficielle et est subdivisée en plusieurs autres couches ayant des caractéristiques biomécaniques propres. Le stratum corneum est la couche la plus externe, c'est celle qui se desquame, et qui est « visible » à l'œil. Cette couche est très rigide, elle permet entre autre de protéger le corps humain des agressions extérieures (chimique, soleil etc). Ses propriétés sont dépendantes de chaque individu, c'est pourquoi les valeurs biomécaniques d'une peau « jeune » sont différentes de celles d'une peau âgée. Une peau âgée est généralement « rigide » (dure) par rapport à une peau jeune qui est « souple » (molle).

Il est connu de l'état de la technique, des équipements utilisant des technologies plus avancées nécessitant des scanners optiques ou la mesure de l'impédance. Les méthodes utilisant ces équipements sont également basées sur des données morphologiques et organisationnelles avec des critères de lésions à l'échelle cellulaire.

Toutefois, leur sensibilité et leur spécificité sont limitées par la taille de la lésion et son emplacement. Ces technologies ne sont pas faciles à utiliser et nécessitent une formation et de l'expérience dans l'interprétation des données.

Une nouvelle génération d'outils connectés est également existante, comme la « dermoscopie numérique » connectée par application smartphone. Les patients peuvent scanner leur peau avec un zoom et des photos de référence sont disponibles pour la comparaison. Ce type d'outil permet aux patients de suivre les lésions suspicieuses, mais ces lésions ne sont pas toujours dangereuses. Ces outils ont principalement une fonction d'analyse basée sur des critères cliniques, qui permettent de détecter uniquement les mélanomes avancés.

### Objet de l'invention

L'invention est definie dans les revendications. La présente demande a pour but de proposer une solution qui réponde à l'un ou plusieurs des problèmes mentionnés ci-dessus.

Ce but peut être atteint grâce à la mise en œuvre d'un dispositif de mesure destiné à être placé au contact d'un tissu, le dispositif de mesure comprenant :
- une surface d'appui destinée à être positionnée au contact du tissu ;
- un indenteur configuré pour occasionner une déformation du tissu, l'indenteur étant configuré pour être déplaçable par rapport à la surface d'appui entre une position d'origine et une position d'indentation dans laquelle le tissu est déformé par l'indenteur ;
- une jauge de contrainte configurée pour mesurer une force de résistance du tissu à la déformation occasionnée par l'indenteur ;
- un capteur de position configuré pour mesurer une profondeur d'indentation représentative d'un déplacement de l'indenteur entre la position d'origine et la position d'indentation.

Les dispositions précédemment décrites permettent de mesurer des caractéristiques biologiques du tissu et notamment la rigidité du tissu qui peut être caractéristique d'au moins une lésion tissulaire si elle est présente au niveau de la zone de prise de mesure.

Le dispositif de mesure peut en outre présenter une ou plusieurs des caractéristiques suivantes, prises seules ou en combinaison.

Selon un mode de réalisation, le tissu est un tissu biologique et plus particulièrement une peau.

Selon un mode de réalisation, le capteur de position comprend un capteur linéaire de position.

Selon un mode de réalisation, la jauge de contrainte a une capacité nominale de ±0,5 N. L'alimentation en énergie électrique est réalisée à une tension de 5V.

De manière générale, la jauge de contrainte est configurée pour convertir une mesure de force en une tension de sortie relative à la mesure de force réalisée.

Selon un mode de réalisation, la position d'origine correspond à la position de l'indenteur lorsqu'il entre en contact avec le tissu.

Selon un mode de réalisation, la mise en contact de l'indenteur avec le tissu est détectée lorsque la jauge de contrainte enregistre une valeur de contrainte supérieure à une valeur seuil, par exemple 0,01 mN. De cette manière, il est possible de détecter la position d'origine automatiquement par une détection d'une valeur de contrainte. Cette position d'origine peut alors servir de point d'origine permettant de d'opérer une superposition entre une courbe témoin théorique avec une courbe réelle mesurée, qui associe un ensemble de mesures de force de résistance à la déformation du tissu avec un ensemble de mesures de profondeurs d'indentation. Ainsi, plus la concordance entre la courbe mesurée et la courbe témoin est forte, plus le résultat de l'analyse sera fiable.

Selon un mode de réalisation, l'indenteur comprend une extrémité distale ayant une forme adaptée à permettre une déformation du tissu sensiblement perpendiculaire à la surface dudit tissu en regard de la surface d'appui.

Selon un mode de réalisation, la mesure de force de résistance à la déformation de tissu est arrêtée mécaniquement lorsque l'extrémité distale de l'indenteur dépasse une profondeur d'indentation de 100 µm. Par exemple, la mesure de force de résistance à la déformation de tissu peut être arrêtée lorsque le capteur de position mesure un déplacement de 100 µm à partir de la position d'origine, ladite position d'origine pouvant correspondre au moment où la jauge de contrainte enregistre une valeur de contrainte supérieure à une valeur seuil.

Selon un mode de réalisation, la mesure de force de résistance à la déformation du tissu peut être arrêtée lorsque la première partie est en butée contre la deuxième partie, ou au bout d'un temps de mesure prédéterminé, ou encore lorsque la mesure est stabilisée.

Selon un mode de réalisation, la jauge de contrainte est configurée pour mesurer la force de résistance à la déformation du tissu selon un axe de mesure, et dans lequel la profondeur d'indentation est mesurée selon l'axe de mesure, ledit axe de mesure étant sensiblement perpendiculaire à la surface d'appui.

Par sensiblement perpendiculaire on entend une direction de l'axe de mesure compris dans un intervalle angulaire de 0° à 5° par rapport à la direction normale au tissu.

Selon un mode de réalisation, le capteur de position comprend un capteur inductif.

Selon un mode de réalisation, le capteur de position peut présenter un pas de 4 µm sur une gamme de 2mm.

Selon un mode de réalisation, le dispositif de mesure comprend un boitier comprenant :
- une première partie sur laquelle prend appui l'indenteur;
- une deuxième partie présentant la surface d'appui et étant reliée à la première partie par l'intermédiaire d'au moins un élément de rappel.

Les dispositions précédemment décrites permettent de stabiliser le dispositif sur le tissu avant la prise de mesure par un utilisateur.

Selon un mode de réalisation, l'élément de rappel comprend au moins un ressort.

Selon un mode de réalisation, le dispositif de mesure comprend une caméra configurée pour collecter une image du tissu représentative du tissu, le dispositif de mesure étant configuré pour communiquer l'image du tissu, la force de résistance à la déformation du tissu et la profondeur d'indentation à destination d'un terminal utilisateur.

Selon un mode de réalisation, le boitier comprend une troisième partie définissant une ouverture, ladite ouverture étant configurée pour délimiter une zone d'intérêt représentative d'une zone à la surface du tissu à partir de laquelle l'image du tissu est collectée.

Selon un mode de réalisation, l'ouverture est comprise dans une chambre noire ménagée dans le boitier, ladite chambre noire étant configurée pour délimiter un volume protégé de la lumière extérieure dans lequel l'image du tissu est collectée. De cette manière, la collecte d'image du tissu par la caméra est reproductible et normalisée.

Selon un mode de réalisation, la jauge de contrainte est configurée pour mesurer une pluralité de force de résistance à la déformation du tissu en un point d'intérêt compris dans la zone d'intérêt, en fonction de la profondeur d'indentation.

Selon un mode de réalisation, le boitier comprend une quatrième partie solidaire de la deuxième partie d'une part et de la troisième partie d'autre part et configurée pour permettre la préhension du dispositif de mesure par une main.

De cette manière, la quatrième partie peut être utilisée comme poignée pour manipuler le dispositif de mesure.

Selon un mode de réalisation, le dispositif de mesure comprend un processeur configuré pour communiquer avec le capteur de position, la jauge de contrainte et la caméra par l'intermédiaire d'un élément convertisseur.

Selon un mode de réalisation le processeur peut communiquer avec le terminal utilisateur par l'intermédiaire d'une antenne.

Selon un mode de réalisation, le processeur peut communiquer avec le terminal utilisateur par connexion filaire.

Selon un mode de réalisation, l'élément convertisseur comprend un convertisseur analogique numérique. Par exemple, ledit convertisseur analogique numérique peut être configuré pour fonctionner à 24 bits.

De manière générale, l'élément convertisseur peut être configuré pour convertir une tension électrique en un signal numérique.

Selon un mode de réalisation, le processeur comprend un microcontrôleur configuré pour contrôler le capteur de position et la jauge de contrainte en temps réel.

Selon un mode de réalisation, le microcontrôleur, le capteur de position et la jauge de contrainte sont contenu dans un volume inférieur à 10050 mm³. De cette manière, les signaux de mesures mesurés par la jauge de contrainte et par le capteur de position sont plus stables, ce qui permet de garantir une mesure plus fiable. Par ailleurs, les dispositions décrites permettent de limiter l'encombrement général et de proposer un dispositif de mesure plus ergonomique.

Selon un mode de réalisation, l'élément convertisseur est configuré pour convertir une pluralité de force de résistance à la déformation du tissu en un premier signal, et pour convertir une pluralité de profondeurs d'indentation en un deuxième signal, le processeur étant configuré pour déterminer, à partir du premier signal et du deuxième signal, une valeur cible de force de résistance à la déformation du tissu correspondant à une profondeur d'indentation prédéterminée.

Selon un mode de réalisation, le processeur est configuré pour communiquer la valeur cible, et l'image du tissu à destination du terminal utilisateur.

Selon une variante, la valeur cible et l'image du tissu peuvent être analysées afin de caractériser une lésion tissulaire, par exemple selon un algorithme prédéterminé.

Selon un mode de réalisation, l'analyse peut être réalisée par un traitement en intelligence artificielle en utilisant un moteur entrainé avec des données de test correspondant à des cas connus. Il est également possible d'utiliser un arbre de décision.

Selon un mode de réalisation, la profondeur d'indentation prédéterminée est comprise entre 90 µm et 110 µm et plus particulièrement sensiblement égale à 100 µm.

Les dispositions précédemment décrites permettent d'effectuer une discrimination entre différent types de lésions cutanées. Pour que cette mesure soit fiable il est notamment primordial de pouvoir garantir que le dispositif de mesure établisse une mesure à 100 µm d'indentation comptés à partir du contact du tissu, car il est possible de réaliser une discrimination entre les lésions cutanées à cette profondeur d'indentation.

De manière avantageuse, la détermination d'une force de résistance à la déformation du tissu en fonction d'une profondeur d'indentation permet de s'affranchir de la force de déformation du tissu, et notamment lorsqu'il existe une variabilité importante entre deux types de tissus, notamment entre des peaux jeunes, ou des peaux âgées. A contrario, dans le cas où la mesure est effectuée par application d'une force donnée, le tissu pourrait être déformé en profondeur d'environ 10% pour une peau âgée, alors qu'il pourrait être déformé en profondeur d'environ 50% pour une peau jeune. Ainsi, l'analyse des données tissulaires n'aurait pas été la même. Par conséquent, et comme décrit précédemment, il est possible pour une profondeur d'indentation de 100 µm, de discriminer un mélanome, d'un carcinome etc., et ce quelque que soient les caractéristiques biomécaniques du tissu.

Selon un mode de réalisation, le dispositif de mesure comprend un dispositif d'éclairage configuré pour éclairer la zone d'intérêt lorsque l'image du tissu est collectée par la caméra.

Selon un mode de réalisation, le dispositif de mesure comprend une lentille convergente et une lentille polarisante.

Selon un mode de réalisation, le dispositif de mesure est alimenté en énergie électrique par l'intermédiaire d'un système de stockage comprenant une batterie.

Selon un mode de réalisation, le dispositif de mesure est alimenté en énergie électrique par l'intermédiaire d'une alimentation filaire.

Selon un mode de réalisation, la première partie, et la troisième partie sont disposées du même côté du boitier.

Le but de la présente demande peut également être atteint grâce à la mise en œuvre d'un procédé d'analyse de données tissulaires concernant un tissu mis en œuvre par ordinateur, le procédé d'analyse de données tissulaires comprenant les étapes suivantes :
- réception en provenance d'une jauge de contrainte d'une mesure d'une force de résistance à la déformation ;
- réception en provenance d'un capteur de position d'une mesure d'une profondeur d'indentation représentative d'un déplacement d'un indenteur par rapport à une position d'origine ;
- association d'une mesure de la profondeur d'indentation et d'une mesure de la force de résistance à la déformation correspondant à la profondeur d'indentation.

Les dispositions précédemment décrites permettent à un utilisateur de recevoir des données mécaniques, comme par exemple la valeur cible, et des données optiques, comme par exemple l'image du tissu sur un terminal utilisateur de manière à réaliser une caractérisation de lésion tissulaire. En particulier, la caractérisation peut correspondre à une analyse de lésion tissulaire de la zone du tissu sur laquelle la mesure a été réalisée.

Le procédé d'analyse de données peut en outre présenter une ou plusieurs des caractéristiques suivantes, prises seules ou en combinaison.

Selon un mode de réalisation, le procédé d'analyse de données tissulaires comprend une étape de détermination d'une valeur cible de force de résistance à la déformation du tissu correspondant à une profondeur d'indentation prédéterminée.

Selon un mode de réalisation, le procédé d'analyse de données tissulaires comprend une étape de réception d'une image du tissu en provenance d'une caméra.

Selon un mode de réalisation, le procédé d'analyse de données tissulaires peut comprendre une étape de transmission de la valeur cible et de l'image du tissu à destination d'un terminal utilisateur. De cette manière, l'analyse des données tissulaires est facilitée par l'image du tissu qui peut aider à représenter une éventuelle lésion tissulaire. En d'autres termes, le procédé d'analyse de données tissulaires permet d'imiter l'acte d'un praticien expert de manière répétitive et fiable. En effet, l'étape de détermination d'une valeur cible de force de résistance à la déformation du tissu correspondant à une profondeur d'indentation prédéterminée peut correspondre à une palpation du tissu par le praticien expert, l'étape de réception d'une image du tissu en provenance d'une caméra peut correspondre à une observation de la surface du tissu par le praticien expert.

Selon un mode de réalisation, le procédé d'analyse de données tissulaires comprend une étape de vérification du niveau de charge d'un système de stockage compris dans un dispositif de mesure d'un type de ceux décrits précédemment. De cette manière, le dispositif de mesure peut être mis sous tension lorsque le niveau de charge du système de stockage est supérieur à un stockage seuil. Par ailleurs, le dispositif de mesure peut être mis dans un état de veille lorsqu'il n'est pas utilisé pendant un temps donné.

Selon un mode de réalisation, le système de stockage comprend une batterie.

Selon un mode de réalisation, l'étape de détermination de la valeur cible est réalisée pour une profondeur d'indentation comprise entre 90 µm et 110 µm, et notamment sensiblement égale à 100 µm.

Selon un mode de réalisation, l'étape de détermination d'une valeur cible de force de résistance à la déformation du tissu correspondant à une profondeur d'indentation prédéterminée est réalisée en moins de 1 s, et notamment en moins de 0.5 s et plus particulièrement en moins de 0.1 s.

Selon un mode de réalisation, le procédé d'analyse de données tissulaires comprend une étape de réception d'une instruction de mesure en provenance d'un utilisateur, l'instruction de mesure étant destiné à :
- déclencher une mesure de force de résistance à la déformation et une mesure de profondeur d'indentation lorsque l'indenteur entre en contact avec le tissu ;
- déclencher une collecte de l'image du tissu lorsque la caméra est disposée à la surface du tissu.

Selon un mode de réalisation, la collecte de l'image du tissu est réalisée au bout d'un temps d'éclairage par un dispositif d'éclairage, par exemple des LEDS. Le temps d'éclairage pouvant être compris entre 1 seconde et 5 secondes et plus particulièrement sensiblement égal à 3 secondes.

Selon un mode de réalisation, le procédé d'analyse de données tissulaires est mis en œuvre par un processeur compris dans un dispositif de mesure d'un type de ceux décrits précédemment.

Selon un mode de réalisation, l'ordinateur comprend une mémoire de données, le procédé d'analyse de données comprenant les étapes suivantes
- enregistrement dans la mémoire de données de la force de résistance à la déformation et de la profondeur d'indentation reçues lors de l'étape de réception de la force de résistance à la déformation et de l'étape de réception de la profondeur d'indentation ;
- détermination d'un ensemble de valeurs de force de résistance à la déformation du tissu mesurées à des profondeurs d'indentation proche de la valeur d'indentation prédéterminée ;
- validation de la valeur cible de force de résistance à la déformation du tissu en fonction dudit ensemble de valeurs de force de résistance à la déformation du tissu.

Par « profondeurs d'indentation proche de la valeur d'indentation prédéterminée », on entend des valeurs de force de résistance à la déformation du tissu mesurée à une profondeur d'indentation comprises dans un intervalle de 10 µm autour de la profondeur d'indentation prédéterminée.

Les dispositions précédemment décrites permettent notamment de vérifier qu'une mesure force de résistance à la déformation du tissu à la profondeur d'indentation prédéterminée est cohérente. Ainsi, les dispositions telles que décrites permettent de s'affranchir d'éventuels artefacts de mesure, d'erreurs de manipulation de la part de l'utilisateur.

Selon un mode de réalisation, le procédé d'analyse de données tissulaires comprend une étape de transmission d'un signal de fin de mesure à destination de l'utilisateur lorsque la profondeur d'indentation mesurée est supérieure à une valeur seuil, la valeur seuil étant strictement supérieure à la profondeur d'indentation prédéterminée.

Selon un mode de réalisation, la valeur seuil est égale à la valeur de la profondeur d'indentation prédéterminée plus 20 µm.

Selon un mode de réalisation, le procédé d'analyse de données tissulaires peut comprendre les étapes suivantes mises en œuvre par le terminal utilisateur :
- réception de l'image du tissu et de la valeur cible en provenance de l'ordinateur ;
- transmission de l'image du tissu et de la valeur cible à destination d'une base de données;
- réception d'une évaluation en provenance de la base de données ;
- transmission de l'évaluation à destination de l'utilisateur.

Les dispositions précédemment décrites permettent de comparer l'image du tissu et la valeur cible à des données comprises dans une base donnée locale ou dans une base de données partagée, de manière à affiner la qualité du diagnostic.

Selon un mode de réalisation, la base de données peut être une base de données locale ou une base de données qui réside sur une plateforme d'infrastructure de cloud computing.

Enfin, le but de la présente demande peut être atteinte par la mise en œuvre d'un produit programme d'ordinateur comprenant des instructions de code agencées pour mettre en œuvre les étapes d'un procédé d'analyse de données du type de celui décrit ci-avant, lorsque ledit programme est exécuté par un processeur.

### Description sommaire des dessins

D'autres aspects, buts, avantages et caractéristiques de la présente demande apparaîtront mieux à la lecture de la description détaillée suivante de modes de réalisation préférés de celle-ci, donnée à titre d'exemple non limitatif, et faite en référence aux dessins annexés sur lesquels :
[Fig. 1] est une vue schématique du dispositif de mesure selon un mode de réalisation.
[Fig. 2] est une vue schématique de la première partie et de la deuxième partie du dispositif de mesure selon un mode de réalisation.
[Fig. 3] est une vue schématique de la troisième partie du dispositif de mesure selon un mode de réalisation.
[Fig. 4] est une vue schématique du dispositif de mesure selon un autre mode de réalisation.
[Fig. 5] est une vue schématique en coupe du dispositif de la figure 4.
[Fig. 6] est une vue schématique du capteur de position du dispositif des figures 4 et 5.
[Fig. 7] est un exemple de séquence illustrant un mode de réalisation du procédé d'analyse de données tissulaires.
[Fig. 8] est un exemple de courbe illustrant l'association d'un ensemble de mesures de force de résistance à **la** déformation du tissu avec un ensemble de mesures de profondeurs d'indentation.
[Fig. 9] est un exemple de deux courbes illustrant l'association d'un ensemble de mesures de force de résistance à la déformation du tissu avec un ensemble de mesures de profondeurs d'indentation.
[Fig. 10] est une vue schématique illustrant un autre mode de réalisation du procédé d'analyse de données tissulaires.

### Description détaillée

Sur les figures et dans la suite de la description, les mêmes références représentent les éléments identiques ou similaires. De plus, les différents éléments ne sont pas représentés à l'échelle de manière à privilégier la clarté des figures. Par ailleurs, les différents modes de réalisation et variantes ne sont pas exclusifs les uns des autres et peuvent être combinés entre eux.

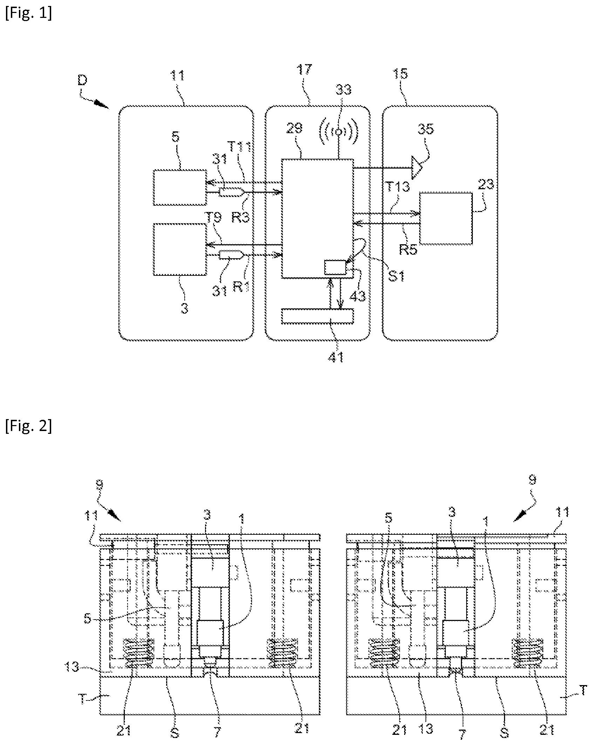

Un premier mode de réalisation est illustré sur la figure 1. Il concerne un dispositif de mesure noté « D » destiné à être placé au contact d'un tissu noté « T ». De manière générale, le tissu T est un tissu T biologique et plus particulièrement une peau.

Selon ce mode de réalisation, le dispositif de mesure D comprend un boitier 9 comprenant une première partie 11, une deuxième partie 13, une troisième partie 15 et une quatrième partie 17.

La première partie 11 comprend une jauge de contrainte 3 et un capteur de position 5.

La deuxième partie 13 présente une surface d'appui notée « S », et destinée à être positionnée au contact du tissu T. La deuxième partie 13 peut être reliée à la première partie 11 par l'intermédiaire d'au moins un élément de rappel 21.

Selon un mode de réalisation, l'élément de rappel 21 peut comprendre un ou plusieurs ressorts.

Les dispositions précédemment décrites permettent de stabiliser le dispositif sur le tissu T avant la prise de mesure par un utilisateur noté « Usr ».

La troisième partie 15 peut comprendre une caméra 23 et un système d'éclairage 35.

Enfin la quatrième partie 17 peut être solidaire de la deuxième partie 13 d'une part et de la troisième partie 15 d'autre part et configurée pour permettre la préhension du dispositif de mesure D par une main. De cette manière, la quatrième partie 17 peut être utilisée comme poignée pour manipuler le dispositif de mesure D.

La première partie 11 et la deuxième partie 13 sont illustrée de manière plus détaillée sur la figure 2, dans deux positions relatives de la première partie 11 par rapport à la deuxième partie 13.

En particulier, un indenteur 1 prend appui sur la première partie 11.

L'indenteur 1 peut être configuré pour occasionner une déformation du tissu T, et pour être déplaçable par rapport à la surface d'appui S entre une position d'origine et une position d'indentation illustrée sur la figure 2B dans laquelle le tissu T est déformé par l'indenteur 1.

La jauge de contrainte 3 peut être configurée pour mesurer une force de résistance du tissu T à la déformation occasionnée par l'indenteur 1, et le capteur de position 5 peut être configuré pour mesurer une profondeur d'indentation représentative d'un déplacement de l'indenteur 1 entre la position d'origine et la position d'indentation.

L'indenteur 1 peut comprendre une extrémité distale 7 ayant une forme adaptée à permettre une déformation du tissu T sensiblement perpendiculaire à la surface dudit tissu T en regard de la surface d'appui S.

Par sensiblement perpendiculaire on entend une direction comprise dans un intervalle angulaire de 0° à 5° par rapport à la direction normale de la surface tissu T.

Selon un mode de réalisation, la position d'origine correspond à la position de l'indenteur 1 lorsqu'il entre en contact avec le tissu T.

Selon un mode de réalisation, la mise en contact de l'indenteur 1 avec le tissu T est détectée lorsque la jauge de contrainte 3 enregistre une valeur de contrainte supérieure à une valeur seuil, par exemple 0,01 mN. De cette manière, il est possible de détecter la position d'origine automatiquement par une détection d'une valeur de contrainte. Cette position d'origine peut alors servir de point d'origine permettant de d'opérer une superposition entre une courbe témoin théorique avec une courbe réelle mesurée, qui associe un ensemble de mesures de force de résistance à la déformation du tissu avec un ensemble de mesures de profondeurs d'indentation. Ainsi, plus la concordance entre la courbe mesurée et la courbe témoin est forte, plus le résultat de l'analyse sera fiable.

Selon un mode de réalisation, la mesure de force de résistance à la déformation de tissu T est arrêtée mécaniquement lorsque l'extrémité distale 7 de l'indenteur 1 dépasse une profondeur d'indentation de 100 µm. Par exemple la mesure de force de résistance à la déformation du tissu T peut être arrêtée lorsque la première partie 11 est en butée contre la deuxième partie 13, ou au bout d'un temps de mesure prédéterminé, ou encore lorsque la mesure est stabilisée.

La jauge de contrainte 3 peut être configurée pour mesurer la force de résistance à la déformation du tissu T selon un axe de mesure. Dans ce cas, la profondeur d'indentation est mesurée selon l'axe de mesure, ledit axe de mesure étant sensiblement perpendiculaire à la surface d'appui S.

Par sensiblement perpendiculaire on entend une direction de l'axe de mesure comprise dans un intervalle angulaire de 0° à 5° par rapport à la direction normale de la surface tissu T.

Selon un mode de réalisation, la jauge de contrainte 3 a une capacité nominale de ±0,5 N. L'alimentation en énergie électrique est réalisée à une tension de 5V.

De manière générale, la jauge de contrainte 3 peut être configurée pour convertir une mesure de force en une tension de sortie relative à la mesure de force réalisée.

Selon un mode de réalisation, la jauge de contrainte 3 est configurée pour mesurer une pluralité de force de résistance à la déformation du tissu T en un point d'intérêt compris dans une zone d'intérêt à la surface du tissu T, en fonction de la profondeur d'indentation.

Selon un mode de réalisation, le capteur de position 5 comprend un capteur linéaire de position.

Selon un mode de réalisation, le capteur de position 5 comprend un capteur inductif.

Selon un mode de réalisation, le capteur de position 5 peut présenter un pas de 4 µm sur une gamme de 2mm.

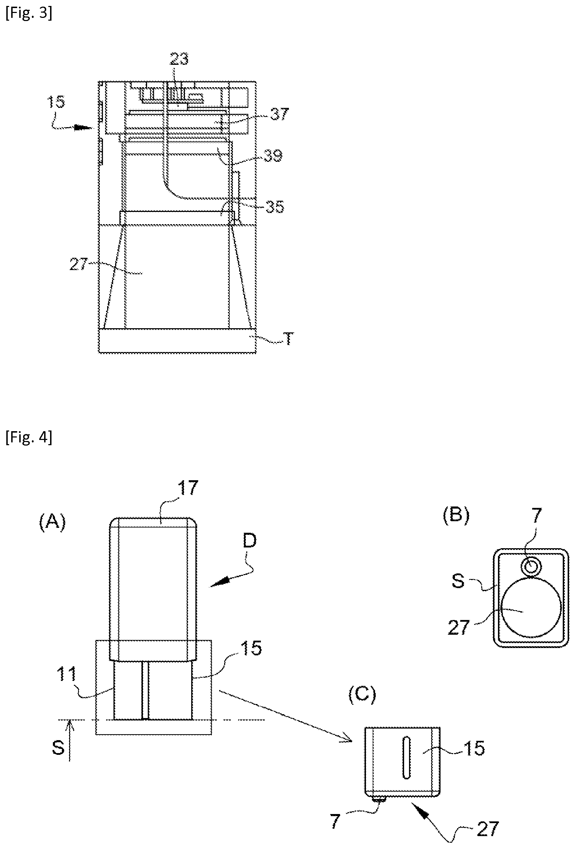

La troisième partie 15 est illustrée sur la figure 3. Elle peut notamment définir une ouverture 27 configurée pour délimiter la zone d'intérêt représentative d'une zone à la surface du tissu T à partir de laquelle une image du tissu T est collectée.

Selon un mode de réalisation, l'ouverture 27 est comprise dans une chambre noire ménagée dans le boitier 9, ladite chambre noire étant configurée pour délimiter un volume protégé de la lumière extérieure dans lequel l'image du tissu T est collectée. De cette manière, la collecte d'image du tissu T par une caméra 23 est reproductible et normalisée.

La troisième partie 15 peut comprendre la caméra 23 configurée pour collecter une image du tissu T représentative du tissu T. Le dispositif de mesure D peut alors être configuré pour communiquer l'image du tissu T, la force de résistance à la déformation du tissu T et la profondeur d'indentation à destination d'un terminal utilisateur 25.

Selon un mode de réalisation, le dispositif de mesure D comprend un dispositif d'éclairage 35 configuré pour éclairer la zone d'intérêt lorsque l'image du tissu T est collectée par la caméra 23.

Le dispositif de mesure D peut également comprendre une lentille convergente 37 et une lentille polarisante 39.

La quatrième partie 17 peut comprendre un processeur 29 configuré pour communiquer avec le capteur de position 5, la jauge de contrainte 3 et la caméra 23 par l'intermédiaire d'un élément convertisseur 31.

Selon un mode de réalisation, le processeur 29 peut communiquer avec le terminal utilisateur 25 par l'intermédiaire d'une antenne 33. Alternativement, le processeur 29 peut communiquer avec le terminal utilisateur 25 par connexion filaire.

Selon un mode de réalisation, le processeur 29 comprend un microcontrôleur configuré pour contrôler le capteur de position 5 et la jauge de contrainte 3 en temps réel. Selon un mode de réalisation, le microcontrôleur, le capteur de position 5 et la jauge de contrainte 3 sont contenu dans un volume inférieur à 10050 mm³. De cette manière, les signaux de mesure mesurés par la jauge de contrainte 3 et par le capteur de position 5 sont plus stables, ce qui permet de garantir une mesure plus fiable. Par ailleurs, les dispositions décrites permettent de limiter l'encombrement général et de proposer un dispositif de mesure D plus ergonomique.

Selon un mode de réalisation, l'élément convertisseur 31 comprend un convertisseur analogique numérique. Par exemple, ledit convertisseur analogique numérique peut être configuré pour fonctionner à 24 bits.

De manière générale, l'élément convertisseur 31 peut être configuré pour convertir une tension électrique en un signal numérique.

Selon un mode de réalisation, l'élément convertisseur 31 est configuré pour convertir une pluralité de force de résistance à la déformation du tissu T en un premier signal, et pour convertir une pluralité de profondeurs d'indentation en un deuxième signal. Le processeur 29 est alors configuré pour déterminer, à partir du premier signal et du deuxième signal, une valeur cible de force de résistance à la déformation du tissu T correspondant à une profondeur d'indentation prédéterminée.

Dans ce cas, le processeur 29 peut être configuré pour communiquer la valeur cible, et l'image du tissu T à destination du terminal utilisateur 25.

Selon une variante, la valeur cible et l'image du tissu peuvent être analysées selon un algorithme afin de caractériser une lésion tissulaire.

En particulier, l'analyse peut être réalisée par un traitement en intelligence artificielle en utilisant un moteur entrainé avec des données de test correspondant à des cas connus. Il est également possible d'utiliser un arbre de décision.

Selon un mode de réalisation, la profondeur d'indentation prédéterminée est comprise entre 90 µm et 110 µm et plus particulièrement sensiblement égale à 100 µm. Les dispositions précédemment décrites permettent d'effectuer une discrimination entre différent types de lésions cutanées. Pour que cette mesure soit fiable il est notamment primordial de pouvoir garantir que le dispositif de mesure D établisse une mesure à 100 µm d'indentation comptés à partir du contact du tissu T, car il est possible de réaliser une discrimination entre les lésions cutanées à cette profondeur d'indentation.

De manière avantageuse, la détermination d'une force de résistance à la déformation du tissu en fonction d'une profondeur d'indentation permet de s'affranchir de la force de déformation du tissu T, et notamment lorsqu'il existe une variabilité importante entre deux types de tissus T, notamment entre des peaux jeunes, ou des peaux âgées. A contrario, dans le cas où la mesure est effectuée par application d'une force donnée, le tissu T pourrait être déformé en profondeur d'environ 10% pour une peau âgée, alors qu'il pourrait être déformé en profondeur d'environ 50% pour une peau jeune. Ainsi, l'analyse des données tissulaires n'aurait pas été la même. Par conséquent, et comme décrit précédemment, il est possible pour une profondeur d'indentation de 100 µm, de discriminer un mélanome, d'un carcinome etc., et ce quelque que soient les caractéristiques biomécaniques du tissu T.

Les dispositions précédemment décrites permettent de mesurer des caractéristiques biologiques du tissu T et notamment la rigidité du tissu T et une image du tissu T qui peuvent être caractéristique d'au moins une lésion tissulaire si elle est présente au niveau de la zone de prise de mesure.

Un autre mode de réalisation du dispositif de mesure D est représenté sur les figures 4 à 6. Selon ce mode de réalisation, et comme représenté sur la figure 4A, la première partie 11, et la troisième partie sont disposées du même côté du boitier 9. Ainsi, la surface d'appui S comprend l'ouverture 27 de la troisième partie 15, comme illustré sur les figure 4B (vue de dessous), et 4C (vue de côté).

Ainsi, l'indenteur 1, le capteur de position 5 et la jauge de contrainte 3 sont disposés du même côté du boitier 9.

De cette manière, la collecte d'image du tissu T par la caméra 23 peut être réalisée simultanément avec une mesure d'une force de résistance à la déformation du tissu par la jauge de contrainte 3. En d'autres termes, il n'est pas nécessaire d'opérer à une rotation du boitier 9 pour réaliser la mesure d'une force de résistance à la déformation du tissu par la jauge de contrainte 3 et la collecte d'image du tissu T par la caméra 23.

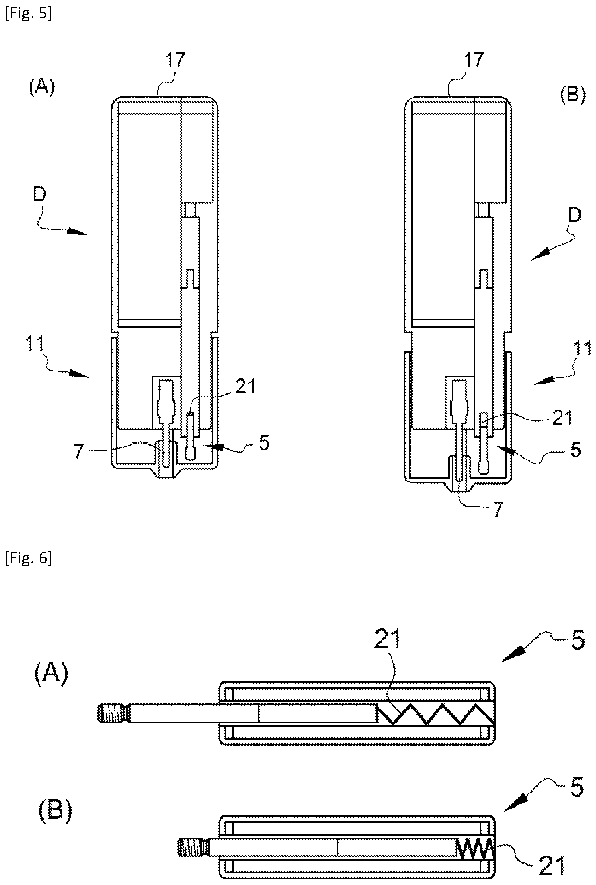

Selon le mode de réalisation représenté sur les figure 4 à 6, l'au moins un élément de rappel 21 est constitué d'un ressort compris sur le capteur de position 5. Ledit capteur de position 5 peut donc prendre appui sur la première partie 11 (figure 5A et 6B) lors de la mesure. L'élément de rappel 21 permet par ailleurs de revenir à une position de repos lorsque le dispositif de mesure D n'effectue pas de mesure (figure 5B et 6A).

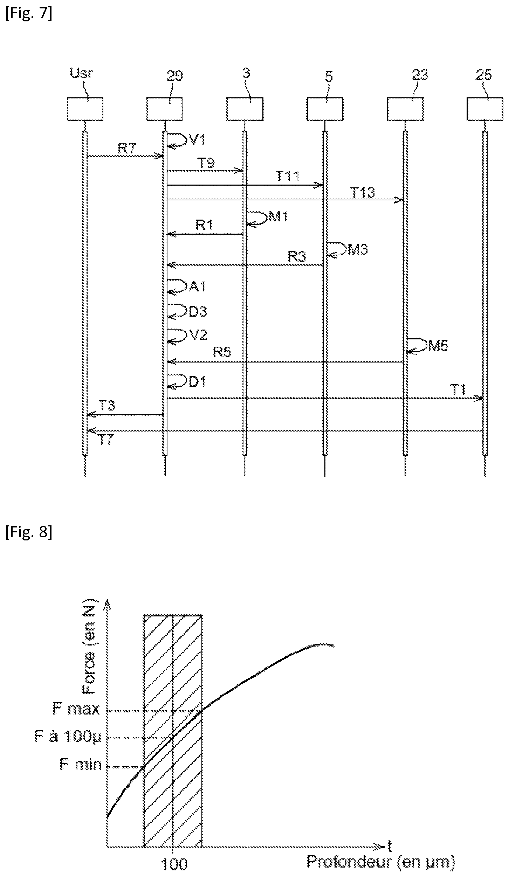

Comme illustré sur la figure 7, un mode de réalisation concerne également un procédé d'analyse de données tissulaires concernant un tissu T, le procédé étant mis en œuvre par ordinateur et par exemple un processeur 29 configuré pour communiquer avec une caméra 23, une jauge de contrainte 3, et un capteur de position 5 par l'intermédiaire d'un élément convertisseur 31, le processeur 29 pouvant être alimenté en énergie électrique par un système de stockage 41. Selon une variante alternative, le processeur 29 du dispositif de mesure D peut être alimenté en énergie électrique par l'intermédiaire d'une alimentation filaire.

En particulier, le système de stockage 41 peut comprendre une batterie.

Selon un mode de réalisation, le procédé d'analyse de données peut comprendre une étape de vérification V1 du niveau de charge du système de stockage 41.

De cette manière, le dispositif de mesure D peut être mis sous tension lorsque le niveau de charge du système de stockage 41 est supérieur à un stockage seuil. Par ailleurs, le dispositif de mesure D peut être mis dans un état de veille lorsqu'il n'est pas utilisé pendant un temps donné.

Selon une variante, le procédé d'analyse de données peut comprendre une étape de réception R7 d'une instruction de mesure en provenance d'un utilisateur Usr, l'instruction de mesure étant destiné à
- déclencher une mesure M1 de force de résistance à la déformation suite à la transmission T9 d'une première instruction de mesure à destination de la jauge de contrainte 3, et à déclencher une mesure M3 de profondeur d'indentation lorsque qu'un indenteur 1 du type de celui précédemment entre en contact avec le tissu T, suite à la transmission T11 d'une deuxième instruction de mesure à destination du capteur de position 5 ;
- déclencher une collecte M5 de l'image du tissu T lorsque la caméra 23 est disposée à la surface du tissu T, suite à la transmission T13 d'une troisième instruction de mesure à destination de la caméra 23.

Selon un mode de réalisation, la collecte M5 de l'image du tissu T est réalisée au bout d'un temps d'éclairage par un dispositif d'éclairage 35, par exemple des LEDS. Le temps d'éclairage pouvant être compris entre 1 seconde et 5 secondes et plus particulièrement sensiblement égal à 3 secondes.

Le procédé d'analyse de données tissulaires comprend notamment les étapes suivantes :
- réception R1 en provenance de la jauge de contrainte 3 d'une mesure de force de résistance à la déformation ;
- réception R3 en provenance du capteur de position 5 d'une mesure de profondeur d'indentation représentative d'un déplacement d'un indenteur 1 par rapport à une position d'origine ;
- association A1 d'une mesure de la profondeur d'indentation et d'une mesure de la force de résistance à la déformation correspondant à la profondeur d'indentation ;
- réception R5 en provenance de la caméra 23 d'une image du tissu T ;
- détermination D1 d'une valeur cible de force de résistance à la déformation du tissu T correspondant à une profondeur d'indentation prédéterminée.

Selon un mode de réalisation la valeur cible est déterminée pour une profondeur d'indentation comprise entre 90 µm et 110 µm, et notamment sensiblement égale à 100 µm.

Selon un mode de réalisation, le processeur 29 peut être apte à communiquer avec un terminal utilisateur 25 par l'intermédiaire d'une antenne 33, le procéder peut alors comprendre une étape de transmission T1 de la valeur cible et de l'image du tissu à destination d'un terminal utilisateur 25 par l'intermédiaire de l'antenne 33. Selon une autre variante non limitative, le processeur 29 peut être apte à communiquer avec un terminal utilisateur 25 par l'intermédiaire d'une connexion filaire.

Les dispositions précédemment décrites permettent à l'utilisateur Usr de recevoir des données mécaniques, comme par exemple la valeur cible, et des données optiques, comme par exemple l'image du tissu T sur un terminal utilisateur 25 de manière à réaliser un diagnostic. En particulier, le diagnostic peut correspondre à une analyse de lésion tissulaire de la zone du tissu T sur laquelle la mesure a été réalisée.

En d'autres termes, le procédé d'analyse de données tissulaires permet d'imiter l'acte d'un praticien expert de manière répétitive et fiable. En effet, l'étape D1 de détermination d'une valeur cible de force de résistance à la déformation du tissu correspondant à une profondeur d'indentation prédéterminée peut correspondre à une palpation du tissu par le praticien expert, l'étape R5 de réception d'une image du tissu en provenance d'une caméra peut correspondre à une observation de la surface du tissu par le praticien expert.

Selon un mode de réalisation particulier pour lequel le processeur 29 comprend une mémoire de données 43, le procédé d'analyse de données peut comprendre en outre les étapes suivantes :
- enregistrement S1 dans la mémoire de données 43 de la force de résistance à la déformation et de la profondeur d'indentation reçues lors de l'étape de réception R1 de la force de résistance à la déformation et de l'étape de réception R3 de la profondeur d'indentation ;
- détermination D3 d'un ensemble de valeurs de force de résistance à la déformation du tissu T mesurées à des profondeurs d'indentation proche de la valeur d'indentation prédéterminée ;
- validation V2 de la valeur cible de force de résistance à la déformation du tissu T en fonction dudit ensemble de valeurs de force de résistance à la déformation du tissu T.

Par « profondeurs d'indentation proche de la valeur d'indentation prédéterminée », on entend des valeurs de force de résistance à la déformation du tissu T mesurées à une profondeur d'indentation comprises dans un intervalle de 10 µm autour de la profondeur d'indentation prédéterminée.

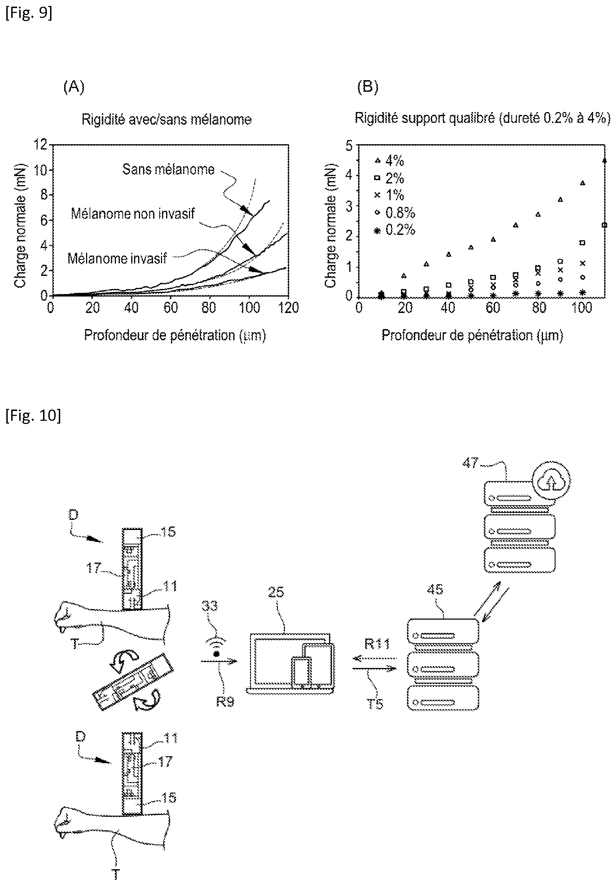

Les figures 8 et 9 illustrent des modes de réalisation pour lesquels chaque mesure d'un ensemble de mesures de force de résistance à la déformation du tissu T est associée à une mesure de profondeur d'indentation d'un ensemble de mesures de profondeur d'indentation. En particulier, la figure 8 illustre les profondeurs d'indentation proche de la valeur d'indentation prédéterminée et les forces correspondantes Fmax et Fmin. La figure 9A illustre notamment comment il est possible de discriminer différents types de mélanomes par la mise en œuvre du dispositif de mesure D selon la présente demande. Cette figure 9A est une représentation graphique des rigidités mesurées par la mise en œuvre du dispositif de mesure D en fonction de différentes lésions (mélanome invasif ou mélanome non invasif) et d'une peau saine (mélanocyte). La figure 9B montre les résultats de rigidité mesurées par la mise en œuvre du dispositif de mesure D selon une matrice standardisée de rigidité croissante (0.2% à 4 %).

Les dispositions précédemment décrites permettent notamment de vérifier qu'une mesure force de résistance à la déformation du tissu T à la profondeur d'indentation prédéterminée est cohérente. Ainsi, les dispositions telles que décrites permettent de s'affranchir des artefacts de mesure et des erreurs de manipulation de la part de l'utilisateur Usr.

Enfin, le procédé d'analyse de données tissulaires peut comprendre une étape de transmission T3 d'un signal de fin de mesure à destination de l'utilisateur Usr lorsque la profondeur d'indentation mesurée est supérieure à une valeur seuil, la valeur seuil étant strictement supérieure à **la** profondeur d'indentation prédéterminée.

Selon un mode de réalisation, **la** valeur seuil est égale à la valeur de la profondeur d'indentation prédéterminée plus 20 µm.

Selon un mode de réalisation illustré sur la figure 10, le procédé d'analyse de données tissulaires peut comprendre les étapes suivantes mises en œuvre par le terminal utilisateur 25 :
- réception R9 de l'image du tissu T et de la valeur cible en provenance du processeur 29 ;
- transmission T5 de l'image du tissu T et de la valeur cible à destination d'une base de données 45, 47 ;
- réception R11 d'une évaluation en provenance de la base de données 45, 47 ;
- transmission T7 de l'évaluation à destination de l'utilisateur Usr.

Les dispositions précédemment décrites permettent de comparer l'image du tissu T et la valeur cible à des données comprises dans une base donnée locale 45 ou dans une base de données partagée 47, de manière à affiner la qualité du diagnostic.

La présente demande concerne enfin un produit programme d'ordinateur comprenant des instructions de code agencées pour mettre en œuvre les étapes d'un procédé d'analyse de données du type de celui décrit précédemment, lorsque ledit programme est exécuté par un processeur 29.

## Revendications

1. Dispositif de mesure (D) destiné à être placé au contact d'un tissu (T), le dispositif de mesure (D) comprenant :
a. un boîtier (9) comprenant une première partie (11), et une deuxième partie (13) reliée à la première partie (11) par l'intermédiaire d'au moins un élément de rappel (21), la deuxième partie (13) comportant une surface d'appui (S) destinée à être positionnée au contact du tissu (T) ;
b. un indenteur (1) prenant appui sur la première partie (11) du boîtier (9) et étant configuré pour occasionner une déformation du tissu (T), l'indenteur (1) étant configuré pour être déplaçable par rapport à la surface d'appui (S) entre une position d'origine et une position d'indentation dans laquelle le tissu (T) est déformé par l'indenteur (1) ;
c. une jauge de contrainte (3) configurée pour mesurer une force de résistance du tissu (T) à la déformation occasionnée par l'indenteur (1), ladite mesure de force de résistance à la déformation du tissu (T) étant arrêtée mécaniquement lorsque la première partie (11) est en butée contre la deuxième partie (13) ;
d. un capteur de position (5) configuré pour mesurer une profondeur d'indentation représentative d'un déplacement de l'indenteur (1) entre la position d'origine et la position d'indentation ;
le dispositif de mesure (D) étant configuré pour détecter automatiquement la position d'origine qui correspond à la position de l'indenteur (1) lorsqu'il entre en contact avec le tissu (T), ce qui est détecté lorsque la jauge de contrainte (3) enregistre une valeur de contrainte supérieure à une valeur seuil.

2. Dispositif de mesure (D) selon la revendication 1, dans lequel la jauge de contrainte (3) est configurée pour mesurer la force de résistance à la déformation du tissu (T) selon un axe de mesure, et dans lequel la profondeur d'indentation est mesurée selon l'axe de mesure, ledit axe de mesure étant sensiblement perpendiculaire à la surface d'appui (S).

3. Dispositif de mesure (D) selon l'une quelconque des revendications 1 ou 2, comprenant une caméra (23) configurée pour collecter une image du tissu (T) représentative du tissu (T), le dispositif de mesure (D) étant configuré pour communiquer l'image du tissu (T), la force de résistance à la déformation du tissu (T) et la profondeur d'indentation à destination d'un terminal utilisateur (25).

4. Dispositif de mesure (D) selon la revendication 3, dans lequel le boitier (9) comprend une troisième partie (15) définissant une ouverture (27), ladite ouverture (27) étant configurée pour délimiter une zone d'intérêt représentative d'une zone à la surface du tissu (T) à partir de laquelle l'image du tissu (T) est collectée.

5. Dispositif de mesure (D) selon la revendication 4 dans lequel la jauge de contrainte (3) est configurée pour mesurer une pluralité de force de résistance à la déformation du tissu (T) en un point d'intérêt compris dans la zone d'intérêt, en fonction de la profondeur d'indentation.

6. Dispositif de mesure (D) selon l'une quelconque des revendications 4 ou 5, dans lequel le boitier (9) comprend une quatrième partie (17) solidaire de la deuxième partie (13) d'une part et de la troisième partie (15) d'autre part et configurée pour permettre la préhension du dispositif de mesure (D) par une main.

7. Dispositif de mesure (D) selon l'une quelconque des revendications 1 à 6, comprenant un processeur (29) configuré pour communiquer avec le capteur de position (5), et la jauge de contrainte (3) par l'intermédiaire d'un élément convertisseur (31) d'une part et destiné à communiquer avec un terminal utilisateur (25) par l'intermédiaire d'une antenne (33) d'autre part, l'élément convertisseur (31) étant configuré pour convertir une pluralité de forces de résistance à la déformations du tissu (T) en un premier signal, et pour convertir une pluralité de profondeurs d'indentation en un deuxième signal, le processeur (29) étant configuré pour déterminer, à partir du premier signal et du deuxième signal, une valeur cible de force de résistance à la déformation du tissu (T) correspondant à une profondeur d'indentation prédéterminée.

8. Dispositif de mesure (D) selon la revendication 7, dans lequel le processeur (29) est configuré pour communiquer la valeur cible, et l'image du tissu (T) à destination du terminal utilisateur (25).

9. Dispositif de mesure (D) selon l'une quelconque des revendications 7 ou 8, dans lequel la profondeur d'indentation prédéterminée est comprise entre 90 µm et 110 µm et plus particulièrement sensiblement égale à 100 µm.

10. Procédé d'analyse de données tissulaires concernant un tissu (T) mis en œuvre par ordinateur, le procédé d'analyse de données tissulaires comprenant les étapes suivantes :
a. réception (R1) en provenance de la jauge de contrainte (3) d'un dispositif de mesure (D) selon l'une quelconque des revendications 1 à 9, d'une mesure d'une force de résistance à la déformation ;
b. réception (R3) en provenance du capteur de position (5) du dispositif de mesure (D), d'une mesure d'une profondeur d'indentation représentative d'un déplacement de l'indenteur (1) par rapport à la position d'origine détectée automatiquement, la position d'origine correspondant à la position de l'indenteur (1) lorsqu'il entre en contact avec le tissu, ce qui est détecté lorsque la jauge de contrainte (3) enregistre une valeur de contrainte supérieure à une valeur seuil ;
c. association (A1) d'une mesure de la profondeur d'indentation et d'une mesure de la force de résistance à la déformation correspondant à la profondeur d'indentation ;
d. détermination (D1) d'une valeur cible de force de résistance à la déformation du tissu (T) correspondant à une profondeur d'indentation prédéterminée.

11. Procédé d'analyse de données tissulaires selon la revendication 10, dans lequel l'étape de détermination (D1) de la valeur cible est réalisée pour une profondeur d'indentation prédéterminée comprise entre 90 µm et 110 µm, et notamment sensiblement égale à 100 µm.

12. Procédé d'analyse de données tissulaires selon l'une quelconque des revendications 10 ou 11, comprenant les étapes suivantes :
- enregistrement (S1) dans une mémoire de données (43) de la force de résistance à la déformation et de la profondeur d'indentation reçues lors de l'étape de réception (R1) de la force de résistance à la déformation et de l'étape de réception (R3) de la profondeur d'indentation ;
- détermination (D3) d'un ensemble de valeurs de force de résistance à la déformation du tissu (T) mesurées à des profondeurs d'indentation proche de la profondeur d'indentation prédéterminée ;
- validation (V2) de la valeur cible de force de résistance à la déformation du tissu (T) en fonction dudit ensemble de valeurs de force de résistance à la déformation du tissu (T) .

## Patentansprüche

1. Messvorrichtung (D), die dazu bestimmt ist, mit einem Gewebe (T) in Kontakt zu gebracht zu werden, wobei die Messvorrichtung (D) Folgendes umfasst:
a. ein Gehäuse (9), das einen ersten Teil (11) und einen zweiten Teil (13) umfasst, der über mindestens ein Rückstellelement (21) mit dem ersten Teil (11) verbunden ist, wobei der zweite Teil (13) eine Auflagefläche (S) aufweist, die dazu bestimmt ist, mit dem Gewebe (T) in Kontakt gebracht zu werden;
b. einen Eindringkörper (1), der auf dem ersten Teil (11) des Gehäuses (9) aufliegt und so konfiguriert ist, dass er eine Verformung des Gewebes (T) verursacht, wobei der Eindringkörper (1) so konfiguriert ist, dass er in Bezug auf die Auflagefläche (S) zwischen einer Ausgangsposition und einer Eindringposition, in der das Gewebe (T) durch den Eindringkörper (1) verformt ist, verschoben werden kann;
c. einen Dehnungsmessstreifen (3), der so konfiguriert ist, dass er eine vom Eindringkörper (1) verursachte Verformungswiderstandskraft des Gewebes (T) misst, wobei die Messung der Verformungswiderstandskraft des Gewebes (T) mechanisch gestoppt wird, wenn der erste Teil (11) gegen den zweiten Teil (13) anstößt;
d. einen Positionssensor (5), der so konfiguriert ist, dass er eine Eindringtiefe misst, die für eine Verschiebung des Eindringkörpers (1) zwischen der Ausgangsposition und der Eindringposition repräsentativ ist;
wobei die Messvorrichtung (D) so konfiguriert ist, dass sie automatisch die Ausgangsposition erkennt, die der Position des Eindringkörpers (1) entspricht, wenn dieser mit dem Gewebe (T) in Kontakt kommt, was erkannt wird, wenn der Dehnungsmessstreifen (3) einen Dehnungswert speichert, der über einem Schwellenwert liegt.

2. Messvorrichtung (D) nach Anspruch 1, wobei der Dehnungsmessstreifen (3) so konfiguriert ist, dass er die Verformungswiderstandskraft des Gewebes (T) entlang einer Messachse misst, und wobei die Eindringtiefe entlang der Messachse gemessen wird, wobei die Messachse im Wesentlichen senkrecht zur Auflagefläche (S) verläuft.

3. Messvorrichtung (D) nach einem der Ansprüche 1 oder 2, umfassend eine Kamera (23), die so konfiguriert ist, dass sie ein Bild des Gewebes (T) erfasst, dass für das Gewebe (T) repräsentativ ist, wobei die Messvorrichtung (D) so konfiguriert ist, dass sie das Bild des Gewebes (T), die Verformungswiderstandskraft des Gewebes (T) und die Eindringtiefe an ein Benutzerterminal (25) übermittelt.

4. Messvorrichtung (D) nach Anspruch 3, wobei das Gehäuse (9) einen dritten Teil (15) umfasst, der eine Öffnung (27) definiert, wobei die Öffnung (27) so konfiguriert ist, dass sie einen Bereich von Interesse abgrenzt, der für einen Bereich an der Oberfläche des Gewebes (T) repräsentativ ist, von dem aus das Bild des Gewebes (T) erfasst wird.

5. Messvorrichtung (D) nach Anspruch 4, wobei der Dehnungsmessstreifen (3) so konfiguriert ist, dass er eine Vielzahl von Widerstandskräften gegen die Verformung des Gewebes (T) an einem Punkt von Interesse innerhalb des Bereichs von Interesse in Abhängigkeit von der Eindringtiefe misst.

6. Messvorrichtung (D) nach einem der Ansprüche 4 oder 5, wobei das Gehäuse (9) einen vierten Teil (17) umfasst, der mit dem zweiten Teil (13) einerseits und dem dritten Teil (15) andererseits fest verbunden und so konfiguriert ist, dass es das Greifen der Messvorrichtung (D) mit einer Hand ermöglicht.

7. Messvorrichtung (D) nach einem der Ansprüche 1 bis 6, umfassend einen Prozessor (29), der so konfiguriert ist, dass er über ein Wandlerelement (31) mit dem Positionssensor (5) und dem Dehnungsmessstreifen (3) einerseits kommuniziert, und dazu bestimmt ist, über eine Antenne (33) mit einem Benutzerterminal (25) andererseits zu kommunizieren, wobei das Wandlerelement (31) so konfiguriert ist, dass er eine Vielzahl von Widerstandskräften gegen die Verformung des Gewebes (T) in ein erstes Signal umwandelt und eine Vielzahl von Eindringtiefen in ein zweites Signal umwandelt, wobei der Prozessor (29) so konfiguriert ist, dass er aus dem ersten Signal und dem zweiten Signal einen Zielwert für die Verformungswiderstandskraft des Gewebes (T) bestimmt, der einer vorbestimmten Eindringtiefe entspricht.

8. Messvorrichtung (D) nach Anspruch 7, wobei der Prozessor (29) so konfiguriert ist, dass er den Zielwert und das Bild des Gewebes (T) an das Benutzerterminal (25) übermittelt.

9. Messvorrichtung (D) nach einem der Ansprüche 7 oder 8, wobei die vorbestimmte Eindringtiefe zwischen 90 µm und 110 µm und insbesondere im Wesentlichen 100 µm beträgt.

10. Verfahren zum computergestützten Analysieren von Gewebedaten hinsichtlich eines Gewebes (T), wobei das Verfahren zum Analysieren von Gewebedaten die folgenden Schritte umfasst:
a. Empfangen (R1) einer Messung einer Verformungswiderstandskraft von dem Dehnungsmessstreifen (3) einer Messvorrichtung (D) nach einem der Ansprüche 1 bis 9;
b. Empfangen (R3) einer Messung einer Eindringtiefe, die für eine Verschiebung des Eindringkörpers (1) in Bezug auf die automatisch erkannte Ausgangsposition repräsentativ ist, von dem Positionssensor (5) der Messvorrichtung (D), wobei die Ausgangsposition der Position des Eindringkörpers (1) entspricht, wenn er mit dem Gewebe in Kontakt kommt, was erkannt wird, wenn der Dehnungsmessstreifen (3) einen Dehnungswert speichert, der über einem Schwellenwert liegt;
c. Kombinieren (A1) einer Messung der Eindringtiefe und einer Messung der Verformungswiderstandskraft, die der Eindringtiefe entspricht;
d. Bestimmen (D1) eines Zielwerts für die Verformungswiderstandskraft des Gewebes (T), der einer vorbestimmten Eindringtiefe entspricht.

11. Verfahren zum Analysieren von Gewebedaten nach Anspruch 10, wobei der Schritt des Bestimmens (D1) des Zielwerts für eine vorbestimmte Eindringtiefe durchgeführt wird, die zwischen 90 µm und 110 µm und insbesondere im Wesentlichen 100 µm beträgt.

12. Verfahren zum Analysieren von Gewebedaten nach einem der Ansprüche 10 oder 11, umfassend die folgenden Schritte:
- Speichern (S1) der Verformungswiderstandskraft und der Eindringtiefe, die beim Schritt des Empfangens (R1) der Verformungswiderstandskraft und beim Schritt des Empfangens (R3) der Eindringtiefe empfangen werden, in einem Datenspeicher (43);
- Bestimmen (D3) eines Satzes von Werten für die Verformungswiderstandskraft des Gewebes (T), gemessen bei Eindringtiefen nahe der vorbestimmten Eindringtiefe;
- Validieren (V2) des Zielwerts für die Verformungswiderstandskraft des Gewebes (T) in Abhängigkeit von dem Satz von Werten für die Verformungswiderstandskraft des Gewebes (T).

## Claims

1. A measurement device (D) intended to be placed in contact with a tissue (T), the measurement device (D) comprising:
a. a housing (9) comprising a first part (11), and a second part (13) connected to the first part (11) through at least one biasing element (21), the second part (13) including a bearing surface (S) intended to be positioned in contact with the tissue (T);
b. an indenter (1) bearing on the first part (11) of the housing (9) and being configured to cause a deformation of the tissue (T), the indenter (1) being configured to be movable relative to the bearing surface (S) between an initial position and an indentation position in which the tissue (T) is deformed by the indenter (1);
c. a strain gauge (3) configured to measure a force of resistance of the tissue (T) to the deformation caused by the indenter (1), said measurement of force of resistance to deformation of the tissue (T) being mechanically stopped when the first part (11) abuts against the second part (13);
d. a position sensor (5) configured to measure an indentation depth representative of a displacement of the indenter (1) between the initial position and the indentation position;
the measurement device (D) being configured to automatically detect the initial position which corresponds to the position of the indenter (1) when it comes into contact with the tissue (T), which is detected when the strain gauge (3) records a strain value greater than a threshold value.

2. The measurement device (D) according to claim 1, wherein the strain gauge (3) is configured to measure the force of resistance to deformation of the tissue (T) along a measurement axis, and wherein the indentation depth is measured along the measurement axis, said measurement axis being substantially perpendicular to the bearing surface (S).

3. The measurement device (D) according to any one of claims 1 or 2, comprising a camera (23) configured to collect an image of the tissue (T) representative of the tissue (T), the measurement device (D) being configured to communicate the image of the tissue (T), the force of resistance to deformation of the tissue (T), and the indentation depth to a user terminal (25).

4. The measurement device (D) according to claim 3, wherein the housing (9) comprises a third part (15) defining an opening (27), said opening (27) being configured to delimit an area of interest representative of an area on the surface of the tissue (T) from which the image of the tissue (T) is collected.

5. The measurement device (D) according to claim 4, wherein the strain gauge (3) is configured to measure a plurality of forces of resistance to deformation of the tissue (T) at a point of interest included in the area of interest, as a function of the indentation depth.

6. The measurement device (D) according to any one of claims 4 or 5, wherein the housing (9) comprises a fourth part (17) secured, on the one hand, to the second part (13) and, on the other hand, to the third part (15), and configured to allow the measurement device (D) to be held by a hand.

7. The measurement device (D) according to any one of claims 1 to 6, comprising a processor (29) configured to communicate with the position sensor (5) and the strain gauge (3) through a converter element (31) on the one hand, and intended to communicate with a user terminal (25) through an antenna (33) on the other hand, the converter element (31) being configured to convert a plurality of forces of resistance to deformation of the tissue (T) into a first signal, and to convert a plurality of indentation depths into a second signal, the processor (29) being configured to determine, from the first signal and the second signal, a target value of force of resistance to deformation of the tissue (T) corresponding to a predetermined indentation depth.

8. The measurement device (D) according to claim 7, wherein the processor (29) is configured to communicate the target value and the image of the tissue (T) to the user terminal (25).

9. The measurement device (D) according to any one of claims 7 or 8, wherein the predetermined indentation depth is comprised between 90 µm and 110 µm, and more particularly substantially equal to 100 µm.

10. A method for analyzing tissue data relating to a tissue (T), implemented by a computer, the method for analyzing tissue data comprising the following steps:
a. receiving (R1), from the strain gauge (3) of a measurement device (D) according to any one of claims 1 to 9, a measurement of a force of resistance to deformation;
b. receiving (R3), from the position sensor (5) of the measurement device (D), a measurement of an indentation depth representative of a displacement of the indenter (1) relative to the automatically detected initial position, the initial position corresponding to the position of the indenter (1) when it comes into contact with the tissue, which is detected when the strain gauge (3) records a strain value greater than a threshold value;
c. associating (A1) a measurement of the indentation depth and a measurement of the force of resistance to deformation corresponding to the indentation depth;
d. determining (D1) a target value of force of resistance to deformation of the tissue (T) corresponding to a predetermined indentation depth.

11. The method for analyzing tissue data according to claim 10, wherein the step (D1) of determining the target value is carried out for a predetermined indentation depth comprised between 90 µm and 110 µm, and in particular substantially equal to 100 µm.

12. The method for analyzing tissue data according to any one of claims 10 or 11, comprising the following steps:
- recording (S1) in a data memory (43) the force of resistance to deformation and the indentation depth received during the step (R1) of receiving the force of resistance to deformation and the step (R3) of receiving the indentation depth;
- determining (D3) a set of values of force of resistance to deformation of the tissue (T) measured at indentation depths close to the predetermined indentation depth;
- validating (V2) the target value of force of resistance to deformation of the tissue (T) as a function of said set of values of force of resistance to deformation of the tissue (T).
